(19) Europäisches Patentamt
European Patent Office
Office européen des brevets

(11) **EP 1 867 360 B1**

(12) **FASCICULE DE BREVET EUROPEEN**

(45) Date de publication et mention
de la délivrance du brevet:
**11.02.2009 Bulletin 2009/07**

(51) Int Cl.:
***A61N 1/365*** *(2006.01)*

(21) Numéro de dépôt: **07290705.8**

(22) Date de dépôt: **07.06.2007**

(54) **Dispositif médical implantable actif, notamment dispositif de stimulation, resynchronisation, défibrillation et/ou cardioversion, comprenant des moyens d'alerte prédictive d'une aggravation de l'état clinique du patient**

Aktives medizinisches Implantat, insbesondere Vorrichtung zur Stimulation, Resynchronisation, Defibrillation und/oder Kardioversion, die Mittel zur prädiktiven Warnung über eine Verschlimmerung des Krankheitszustands des Patienten umfasst

Active implantable medical device, in particular a stimulation, resynchronisation, defibrillation and/or cardioversion device comprising predictive means for warning of an aggravation of the patient's clinical status

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **15.06.2006 FR 0605322**

(43) Date de publication de la demande:
**19.12.2007 Bulletin 2007/51**

(73) Titulaire: **ELA MEDICAL**
**92541 Montrouge (FR)**

(72) Inventeur: **Casset, Cyrille**
**91130 Ris Orangis (FR)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
**EP-A- 0 804 939          EP-A- 0 966 987**
**EP-A- 1 533 001          US-A1- 2003 040 776**

**Description**

L'invention concerne les « dispositifs médicaux implantables actifs » tels que définis par la directive 90/385/CEE du 20 juin 1990 du Conseil des communautés européennes, notamment les stimulateurs cardiaques, dispositifs de resynchronisation, cardioverteurs et/ou défibrillateurs destinés au traitement des troubles du rythme cardiaque, ou encore les implants actifs à visée purement diagnostique.

Elle concerne plus précisément ces dispositifs dont le fonctionnement est asservi à des paramètres recueillis par des capteurs permettant d'évaluer les besoins métaboliques du porteur de l'appareil ainsi que son niveau courant d'activité.

Ces dispositifs comportent deux capteurs de nature différente, à savoir un capteur de mesure d'un paramètre corporel à prépondérance physiologique, c'est-à-dire d'un paramètre représentatif des besoins métaboliques du patient, et un capteur de mesure d'un paramètre corporel à prépondérance physique.

La fréquence cardiaque est également utilisée pour le contrôle du stimulateur, mais ce paramètre n'est pas directement représentatif des besoins métaboliques du patient ni de son activité physique instantanée.

Dans la suite on prendra comme exemple de capteur physiologique un capteur de ventilation-minute (MV), car il s'agit du cas le plus courant. Mais ce choix n'a aucun caractère limitatif et d'autres types de capteurs produisant un signal représentatif des besoins métaboliques du patient peuvent être également utilisés, notamment un capteur de saturation d'oxygène dans le sang. De même, on prendra comme exemple de capteur physique un capteur d'accélération (G), qui est le capteur plus couramment utilisé, mais d'autres types de capteurs sont envisageables pour notamment détecter les mouvements du patient.

De façon générale, le capteur physique est caractérisé par un temps de réponse plus court que le capteur physiologique, de manière à permettre une détection très rapide des efforts de courte durée avant même que le paramètre physiologique, de variation plus lente, n'ait évolué de manière significative.

Le EP-A-0 750 920 (ELA Medical) décrit un tel dispositif asservi à deux capteurs, qui opère une sélection de l'un ou l'autre capteur de manière à retenir celui qui donne le signal le plus pertinent à un instant donné. Le EP-A-0 919 255 (ELA Medical) prévoit, quant à lui, d"asservir le dispositif à une combinaison des signaux délivrés par les deux capteurs.

Les signaux délivrés par ces capteurs peuvent être utilisés pour contrôler l'application d'impulsions de stimulation au patient, dans le cadre d'une thérapie antibradycardique ou pour empêcher la survenue de syncopes d'origine vaso-vagale.

Les EP-A-1 533 001 (ELA Medical) et US-A-2003/040776 décrivent des dispositifs conçus pour de telles applications.

Une autre application des signaux délivrés par les deux capteurs physique et physiologique consiste à opérer un diagnostic de l'insuffisance cardiaque, notamment pour agir de façon appropriée et préventive sur la programmation du stimulateur.

L'invention concerne spécifiquement ces dispositifs pourvus de moyens de diagnostic de l'insuffisance cardiaque.

Ainsi, le EP-A-0 966 987 (ELA Medical) propose un dispositif permettant de suivre l'évolution du patient au cours du temps de manière à donner une représentation adéquate des besoins métaboliques réels de celui-ci, c'est-à-dire prenant en compte le niveau réel d'activité du patient. Ce dispositif adapte son fonctionnement en cas d'aggravation ou d'amélioration de la situation du patient, notamment par reprogrammation de certaines de ses fonctions, de manière à prendre en compte l'évolution de l'état du patient et s'adapter au degré de décompensation cardiaque effectif de celui-ci.

Le point de départ de l'invention réside dans l'observation de patients qui, même appareillés avec de tels dispositifs perfectionnés, adaptent leur activité quotidienne aux modifications de leur état clinique, avec une incidence sur leur niveau d'activité et, dans un certain nombre de cas, sur leur état respiratoire.

En effet, les modifications cliniques pouvant être asymptomatiques, il est courant que le patient adapte inconsciemment son activité à sont état clinique : les premières crises d'insuffisance cardiaque apparaissant à l'effort, ceci conduit le patient à réduire son activité pour éviter la survenue de telles crises. Ensuite, les symptômes n'apparaissent plus, le patient ayant modifié son comportement pour les éviter, mais la pathologie continue à progresser. Et ce n'est que lorsque l'insuffisance cardiaque gênera le patient même au repos qu'il ira consulter ou, dans les cas extrêmes, devra être hospitalisé en urgence.

En résumé, du fait de cette auto-adaptation, l'absence de symptôme ressenti cause un délai important entre le début des modifications cliniques et le diagnostic de ces modifications, souvent trop tardif.

Il a été proposé d'exploiter les informations issues des deux capteurs physiologique et physique de manière à évaluer l'état clinique réel du patient et l'évolution de sa pathologie même en l'absence de symptômes ressentis, et même si le patient adapte inconsciemment son comportement à l'évolution de son état clinique (c'est-à-dire même s'il réduit son activité pour échapper aux crises d'insuffisance cardiaque).

Pour ce faire, le dispositif évalue et met à jour, par exemple quotidiennement, un ou plusieurs index reflétant l'état clinique du patient. L'évolution de ces index est analysée par le dispositif, qui peut notamment délivrer un signal d'alerte préventive lorsque sont vérifiés un certain nombre de critères laissant suspecter une aggravation de l'état clinique du patient même en l'absence de symptôme grave. Cette alerte permet de prendre au plus vite les mesures appropriées et évite ainsi le déclenchement imprévu d'une crise à brève échéance.

Une difficulté rencontrée avec ce type d'analyse réside dans le paramétrage des différents critères de déclenchement de l'alerte. Les valeurs prises par les différents index d'état clinique sont confrontées quotidiennement à une batterie de ces critères, comprenant des paramètres fixes de type seuil, des paramètres incrémentaux tels que des pourcentages d'augmentation minima ou maxima, des métarègles analysant l'évolution des index et de leur combinaison sur plusieurs jours, etc. Le paramétrage de cette batterie de critères (règles et métarègles) peut être modifié pour augmenter la sensibilité de déclenchement de l'alerte ou, inversement, pour une plus grande spécificité de ce déclenchement.

Dans la présente description, on considérera que la sensibilité et la spécificité (c'est-à-dire la sélectivité) sont deux notions antagonistes, dans la mesure où une augmentation de sensibilité s'accompagne automatiquement d'une moindre spécificité - avec corrélativement une augmentation des risques de fausses alertes -, et réciproquement une augmentation de la spécificité se fera au détriment de la sensibilité - avec le risque de ne pas déclencher d'alerte dans des cas critiques. En d'autres termes, plus les critères d'alerte sont stricts, plus les cas de faux positifs sont rares mais, en contrepartie, les risques de faux négatifs augmentent. Inversement, si l'on veut réduire les cas de fausses alertes, on risque de manquer des alertes justifiées.

Le point de départ de la présente invention réside dans la constatation de ce que, avec les techniques connues, le paramétrage des critères de déclenchement d'alerte implique toujours de faire un compromis entre sensibilité et spécificité.

Au surplus, compte tenu de la multiplicité des paramètres intervenant dans le déclenchement de l'alerte, il est difficile pour le praticien d'évaluer le degré réel de sensibilité et de spécificité du dispositif. Le comportement du dispositif face à une situation donnée sera ainsi difficile à prévoir, et même le praticien pourra avoir du mal à déterminer la pertinence des réactions du dispositif.

Enfin, l'alerte délivrée étant du type « tout ou rien » et compte tenu des risques de faux positifs, le patient risque de s'alarmer inutilement en cas de déclenchement d'une alerte, qui ne lui donne pas de moyen d'évaluer le degré réel du risque signalé. Or certaines évolutions de la pathologie nécessitent une action sans délai (retour d'urgence du patient à un contrôle médical), tandis que d'autres révèlent seulement une dégradation lente de l'état du patient, avec un risque moindre, ne nécessitant pas une hospitalisation en urgence.

Le dispositif de l'invention est un dispositif du type général décrit par le EP-A-0 966 987 précité, comportant des moyens de diagnostic de l'insuffisance cardiaque tels que ceux énoncés dans le préambule de la revendication 1.

Pour résoudre les problèmes et pallier les limitations que l'on a indiquées plus haut, les moyens de diagnostic de l'insuffisance cardiaque du dispositif de l'invention comprennent en outre les moyens énoncés dans la partie caractérisante de cette revendication 1.

Les sous-revendications visent des formes de mise en oeuvre particulières de l'invention.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 est un schéma par blocs montrant les différentes fonctions du dispositif impliquées dans la détermination des index d'état clinique et dans le diagnostic prédictif.

La figure 2 illustre la manière dont sont prises en compte les variations du signal d'accélération par le dispositif de l'invention.

La figure 3 illustre les variations de la ventilation-minute moyenne en activité, sur une période de plusieurs semaines.

La figure 4 illustre la fonction de transfert pour la détermination des index d'état clinique relatifs à l'accélération et au temps passé en activité.

La figure 5 illustre la fonction de transfert pour la détermination des index d'état clinique relatifs à la ventilation-minute en activité et au repos.

La figure 6 illustre sous forme graphique la dualité sensibilité/spécificité, avec les différents choix possibles de batteries de critères d'analyse.

La figure 7 illustre, sous forme de schéma par blocs, les différentes fonctions impliquées dans la technique d'analyse selon l'invention.

La figure 8 est une table de vérité indiquant les différents niveaux d'alerte produits par le dispositif de l'invention en fonction des valeurs respectives que peuvent prendre les signaux d'alerte d'état clinique et de contractilité cardiaque.

La figure 9 illustre la répartition dans un espace bidimensionnel des valeurs quotidiennes prises par les index d'état clinique et de contractilité cardiaque, de manière illustrative des différentes zones de risques et de la manière dont le dispositif de l'invention réagit en fonction de l'évolution quotidienne de ces index.

On va maintenant décrire un exemple de réalisation du dispositif de l'invention. En ce qui concerne ses aspects logiciels, l'invention peut être mise en oeuvre par une programmation appropriée du logiciel de commande d'un stimulateur asservi connu.

L'invention peut notamment être appliquée aux dispositifs implantables commercialisés par ELA Médical, Montrouge, France tels que les appareils *Symphony* et *ELA Rhapsody*. Il s'agit de dispositifs à microprocesseur programmable comportant des circuits pour recevoir, mettre en forme et traiter des signaux électriques recueillis par des électrodes et divers capteurs. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

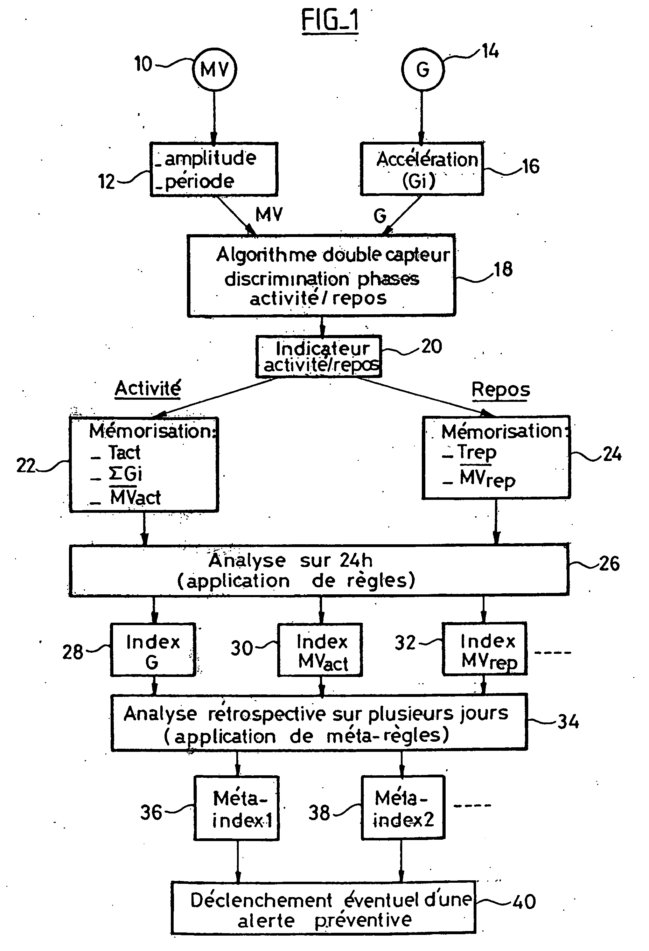

Comme illustré figure 1, le dispositif comporte un capteur 10 délivrant un signal représentatif des besoins métaboliques du patient, typiquement un signal d'impédance transthoracique dont l'analyse des variations périodiques (amplitudes et périodes successives) est opérée par le bloc 12 qui délivre un signal de ventilation-minute (MV). Le dispositif comporte également un capteur physique permettant de détecter les mouvements du patient, typiquement un capteur d'accélération 14 associé à un circuit d'échantillonnage 16 délivrant une succession d'échantillons numérisés $G_i$, avec un pas i = 125 ms par exemple.

À partir des informations MV et G délivrées concurremment, le dispositif opère un asservissement de type « double capteur » (bloc 18) comme décrit dans les EP-A-0 750 920 et EP-A-0 919 255 antérieurs précités, notamment un asservissement de la fréquence de stimulation et une adaptation éventuelle des paramètres de fonctionnement. Cette fonction d'asservissement proprement dite n'entre toutefois pas dans le cadre de l'invention et ne sera pas décrite en détail.

L'algorithme d'asservissement présente toutefois l'avantage de posséder une fonction de discrimination des phases d'activité et de repos du patient (bloc 18) à partir des indications instantanées par les capteurs MV et G, dont il résulte un indicateur 20 pouvant prendre au moins deux valeurs « activité » et « repos » (d'autres valeurs étant également possibles, par exemple « récupération après effort » qui sera assimilé à une phase d'activité, ou « sommeil » qui n'est qu'un cas particulier de phase de repos).

Le dispositif mémorise, de façon distincte pour les phases d'activité et de repos, les données fournies par les capteurs MV et G.

Pour les paramètres caractéristiques en phase d'activité, le dispositif mémorise (bloc 22) :

- le temps passé en activité sur les dernières 24 heures (Tact),
- la somme des mesures du capteur G en phase d'activité ΣG, et
- la ventilation-minute moyenne (MVact) pendant les phases d'activité, mesurée sur les dernières 24 heures.

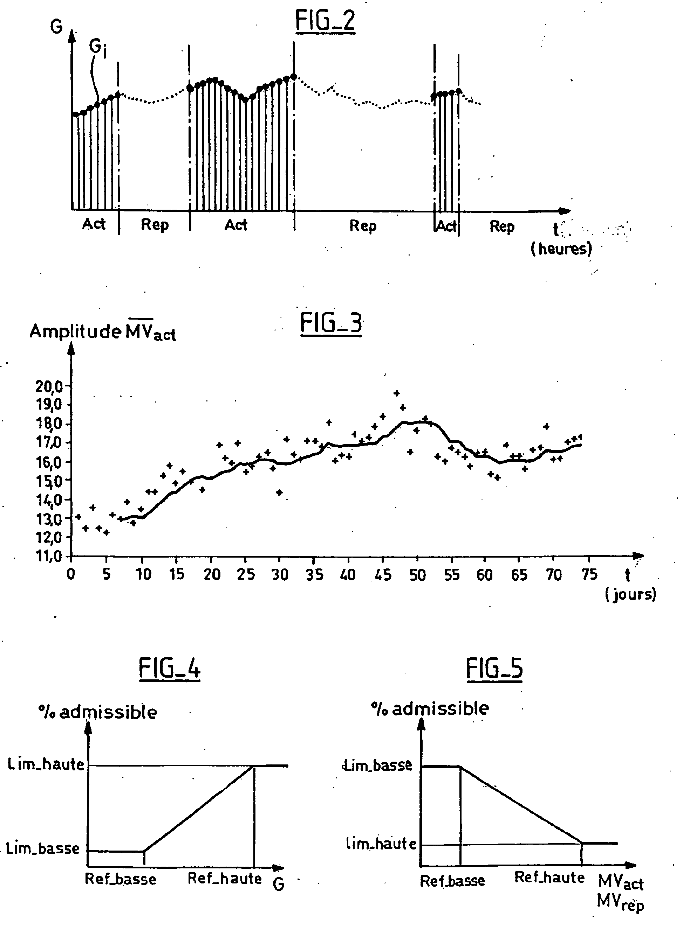

La figure 2 illustre plus précisément la manière dont est obtenue et mise à jour la donnée $ΣG_i$. Comme on l'a indiqué plus haut, le capteur G fournit une série d'échantillons numérisés $G_i$ à intervalles de par exemple i = 125 ms, dont la variation au fil du temps est illustrée sur la figure 2. Par ailleurs, l'indicateur 20 permet de distinguer les périodes d'activité (Act) et de repos (Rep). Le dispositif procède à la sommation des valeurs $G_i$ des échantillons depuis les dernières 24 heures, mais en inhibant cette sommation pendant les périodes de repos, en ne sommant donc que des valeurs correspondant à des périodes d'activité.

Le paramètre MVact, quant à lui, est une valeur moyenne de la ventilation-minute MV lors des périodes d'activité. La figure 3 illustre par exemple les variations de ce paramètre MVact au cours du temps sur une durée de plusieurs semaines : les croix indiquent les valeurs calculées quotidiennement et mémorisées, le trait continu indiquant une moyenne mobile sur 7 jours.

Pour les paramètres caractéristiques en phase de repos, le dispositif mémorise (bloc 24) :

- le temps passé en repos sur les dernières 24 heures (Trep), et
- la ventilation-minute moyenne (MVrep) pendant les phases de repos, mesurée sur les dernières 24 heures.

Le paramètre MVrep est une valeur moyenne de la ventilation-minute MV lors des périodes de repos.

Ces diverses informations mémorisées et mises à jour dans les tableaux de valeurs des deux blocs 22 et 24 sont ensuite soumises à une analyse (bloc 26) consistant à appliquer un certain nombre de règles d'inférence donnant une série d'index d'état clinique, tel que notamment :

- un index 28 lié à l'accélération (index G),
- un index 30 lié à la ventilation-minute en activité (index MVact),
- un index 32 lié à la ventilation-minute au repos (index MVrep).

Cette analyse est opérée par exemple chaque jour, avec mise à jour quotidienne des divers index d'état clinique.

Plusieurs méthodes d'analyse sont possibles. Une première méthode consiste à comparer les valeurs mémorisées à diverses références fixes, et à détecter le franchissement de seuils hauts et/ou bas.

Une autre méthode avantageuse, que l'on va décrire ci-dessous, consiste à analyser les variations des données d'un jour au suivant (ou sur un autre intervalle, par exemple d'une semaine à la suivante, etc.) et comparer cette variation à une valeur de référence correspondant à un pourcentage de variation physiologiquement admissible compte tenu de l'état clinique du patient. Le franchissement de cette limite révèlera la dégradation de l'état du patient à l'égard du critère correspondant (activité, ventilation basale, etc.)

On va décrire en détail par exemple la règle d'inférence correspondant au critère G, les règles correspondant aux autres critères étant appliquées de façon comparable.

Les paramètres mémorisés relatifs aux phases d'activité contiennent une série de valeurs *Somme_G(i),* le pas i correspondant ici à un intervalle d'une journée.

Plutôt que d'appliquer la règle d'inférence à la valeur quotidienne isolée, on calcule préalablement une moyenne mobile sur une semaine de manière à pondérer les variations d'activité d'un jour à l'autre, moyenne que l'on peut exprimer de la manière suivante:

$$M7G(i) = (1/7) * \sum_{k=i-6}^{i} somme\_G(k)$$

La règle relative au paramètre G sera désignée "règle R1", et le résultat de son application sera un indicateur pouvant prendre trois valeurs, représentatives de l'évolution de l'état clinique du patient au regard de ce paramètre G : +1 (amélioration), -1 (dégradation), ou 0 (stabilité).

En effet, l'augmentation de l'activité est un élément favorable dans le tableau clinique du patient, ce qui justifie le score +1, tandis qu'une réduction de ses activités est au contraire un élément défavorable sur le long terme.

Ces valeurs sont attribuées de la manière suivante :

Règle R1 :

Si $M7G(i) > M7G(i-6)*[1 + \alpha(M7G(i))]$, alors R1 (i) = +1 ;
Si $M7G(i) < M7G(i-6)*[1 + \alpha(M7G(i))]$, alors R1 (i) = -1 ; R1 (i) = 0 sinon

$\alpha$ (M7G(i)) étant un pourcentage calculé à partir d'une fonction de transfert dépendant de la valeur courante du critère considéré.

L'intérêt d'un facteur $\alpha$ non constant est lié au besoin de tenir compte des résultats précédents pour qualifier l'amélioration ou la dégradation du résultat : ainsi, pour un patient qui a une activité très faible une augmentation d'activité, même modeste, sera un élément très favorable, ce qui n'est pas nécessairement le cas pour un patient ayant une activité soutenue et en augmentation régulière.

La figure 4 illustre la fonction de transfert donnant le taux d'augmentation admissible (ci-après *Seuilparamètre*) en fonction de la valeur du paramètre considéré.

Dans l'exemple indiqué plus haut du paramètre G, la fonction de transfert est définie de la manière suivante :

*Seuilparamètre*(M7(i)) = *Limite_basse* si M7(i) < *Réf basse ;*
*Seuilparamètre*(M7(i)) = *Limite_haute* si M7(i) > *Réf haute ;*
*Seuilparamètre*(M7(i)) = A*M7(i)+B,

A, B étant la pente et l'ordonnée à l'origine de la droite passant par les points *(Réf basse, Limite_basse)* et *(Réf_haute, Limite_haute).*

Le principe de cette fonction de transfert est également applicable, *mutatis mutandis,* à la durée passée en activité Tact.

En revanche, pour le paramètre de ventilation, en activité (MVact) ou au repos (MVrep), le sens de variation de la fonction de transfert doit être inversé, comme illustré sur la figure 5. En effet, dans le cas où un patient est à une ventilation de repos élevée, la plus petite augmentation suffit à le gêner fortement, alors qu'à plus faible ventilation, il possède encore une marge d'augmentation importante.

Les résultats ainsi obtenus par l'application des diverses règles peuvent faire l'objet d'un diagnostic plus poussé, par application de métarègles, permettant de définir un ou plusieurs index de niveau supérieur, ou « méta-index ».

Ces méta-index sont déterminés (bloc 34) par une analyse rétrospective des valeurs successives prises par les différents index.

Ces métarègles peuvent être élaborées de diverses manières, et on en donnera ci-dessous simplement un exemple, consistant à définir une limite sur plusieurs jours (p jours) sur lesquels il faut qu'une règle R délivre un nombre minimum de valeurs positives de l'index correspondant (ce minimum étant paramétré à la valeur *Seuil_MR*) :

Métarègle MR (une valeur +1 du méta-index étant représentative d'une aggravation significative de l'état du patient) :

$$MR(i) = \sum_{k=i-p+1}^{i} R(k) \text{, avec p entier naturel}$$

Si MR(i) > *Seuil_MR*, alors MR(i) = +1 ;

MR(i) = 0 sinon

Cette métarègle prend en compte le fait d'avoir des valeurs d'index nulles ou négatives: en effet, des valeurs nulles n'augmentent pas la valeur MR(i) du méta-index, tandis que des valeurs négatives ont pour effet de « compenser » les valeurs positives précédemment prises.

On obtient ainsi un ou plusieurs méta-index 36, 38, ... reflétant l'évolution de l'un des paramètres G, MVact, MVrep, ... ou d'une combinaison de ces divers paramètres.

Une alerte peut être déclenchée en fonction des résultats produits par les règles et métarègles (bloc 40). Cette alerte permet d'anticiper de façon extrêmement précoce, avant tout symptôme grave, l'apparition d'un évènement tel qu'une crise d'insuffisance cardiaque afin de prendre sans attendre les mesures nécessaires pour empêcher la survenue de cette crise, ou à tout le moins en réduire les effets, bien avant que le patient n'ait fait appel de lui-même à son praticien ou à un service d'urgence.

Mais, concrètement, le déclenchement ou le non-déclenchement de l'alerte peut être influencé par des évènements ou situations particuliers qui ne sont pas pris en compte par la technique d'analyse décrite ci-dessus.

En premier lieu, si la décompensation cardiaque a bien un effet sur l'activité et/ou l'état ventilatoire du patient - ce que détecte bien le dispositif - d'autres évènements peuvent également modifier ces paramètres cliniques, notamment des situations pathologiques non liées à la décompensation cardiaque telles que cancer évolutif, pneumonie, grippe, ... L'analyse n'est donc pas exclusivement spécifique à la décompensation cardiaque et peut être influencée par d'autres pathologies de nature totalement différente.

En second lieu, la constante de temps de l'analyse de l'activité et/ou de l'état ventilatoire du patient, et le déclenchement éventuel d'une alerte, sont relativement importants, typiquement de plusieurs jours. Or, dans certaines situations de décompensation cardiaque brutale (en 24 heures par exemple), le temps de réaction sera trop long pour qu'une alerte soit produite à temps. En effet, si l'analyse que l'on vient de décrire est bien adaptée aux situations de dégradations lentes, elle ne prend pas suffisamment en compte les situations de crise impliquant une baisse rapide du tonus cardiaque.

L'idée de base de la présente invention est de remédier à ces deux inconvénients en combinant l'analyse des paramètres physiologiques et physiques (ventilation-minute MV et accélération G) à l'analyse des variations d'un signal hémodynamique, représentatif de la contractilité du myocarde et donc très spécifique à la décompensation cardiaque.

De façon caractéristique, le dispositif de l'invention comporte à cet effet outre les capteurs physiologique (MV) et physique (G), un capteur hémodynamique permettant d'estimer en outre les variations de contractilité, qui sont corrélées aux augmentations de pression sanguine.

Cependant, par rapport aux signaux MV ou G, les informations délivrées par les capteurs hémodynamiques présentent l'inconvénient d'être beaucoup plus bruitées et sensibles à de nombreux facteurs externes tels que fréquence cardiaque, activité en cours, médication, etc.

Pour pallier cet inconvénient, le dispositif de l'invention opère un contrôle croisé entre, d'une part, les informations issues des capteurs MV et G (représentatives de l'état clinique du patient) et, d'autre part, celles issues du capteur hémodynamique (représentatives de la contractilité cardiaque). Ce contrôle croisé permet de délivrer une alerte pertinente, adaptée à chaque cas clinique, avec :

- une meilleure discrimination entre les alertes concernant des décompensations cardiaques et les alertes produites par d'autres causes (grippe, handicap physique, changement brusque d'habitude,...) et
- réduction significative de la constante de temps de déclenchement de l'alerte, le dispositif étant apte à réagir de façon efficace en cas de baisse soudaine du tonus cardiaque.

Par ailleurs, pour améliorer notablement le rapport sensibilité/spécificité (c'est-à-dire réduire à la fois les faux positifs et les faux négatifs), le dispositif peut très avantageusement utiliser non plus une batterie de critères mais au moins deux batteries de critères distincts (par « batterie de critères », on entendra l'ensemble des règles et, le cas échéant, des métarègles qui concourent à la production, ou non, d'un signal d'alerte).

En appliquant ces deux (ou plus) batteries de critères aux signaux recueillis et en comparant les résultats obtenus (alerte/pas d'alerte) pour chacune des batteries de critères, le dispositif pourra produire une action encore mieux adaptée à la situation clinique effective du patient.

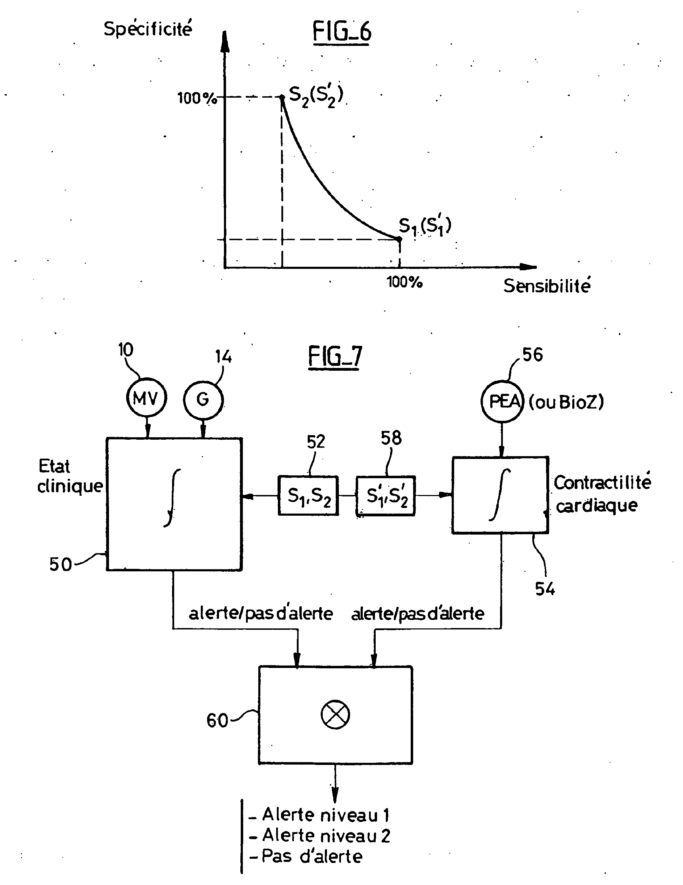

La figure 6 illustre la dualité des deux paramètres spécificité/sensibilité dans le choix des batteries de critères : pour l'analyse de l'état clinique, on peut ainsi choisir une batterie de critères S1 présentant une sensibilité maximale et une faible spécificité, et une batterie de critères S2 présentant, inversement, une spécificité maximale mais une sensibilité moindre. Il est également possible de choisir tout autre couple de batteries de critères {S1, S2} situé entre ces deux extrêmes.

De la même façon, pour l'analyse de la contractilité cardiaque, on pourra prendre deux batteries de critères S'1 à forte sensibilité (et donc à faible spécificité) et S'2 à forte spécificité (et donc faible sensibilité).

La figure 7 illustre schématiquement la mise en oeuvre de la technique d'analyse croisée selon l'invention.

Le bloc 50 représente une fonction de transfert correspondant à l'ensemble des étapes décrites en référence à la figure 1. Cette fonction de transfert analyse l'état clinique du patient à partir des informations délivrées par les capteurs physiologique (MV) 10 et physique (G) 14.

Par application de deux batteries de critères différentes {S1, S2} schématisés en 52, la fonction de transfert du bloc 50 produit, ou ne produit pas, un signal d'alerte en sortie, et ce distinctement pour chacune des deux batteries de critères S1 et S2.

De la même façon, une fonction de transfert représentée par le bloc 54 évalue la contractilité cardiaque à partir du signal délivré par un capteur hémodynamique 56.

Par application de deux batteries de critères différentes {S'1, S'2} schématisés en 58, la fonction de transfert du bloc 52 produit, ou ne produit pas, un signal d'alerte en sortie, et ce distinctement pour chacune des deux batteries de critères S'1 et S'2.

Le capteur hémodynamique 56 est avantageusement un capteur d'accélération endocardiaque de type PEA *(Peak Endocardial Acceleration)* tel que celui décrit par exemple dans les EP-A 0 515 319, EP-A 0 582 162 ou EP-A 0 655 260 (tous trois au nom de Sorin Biomedica Cardio SpA) . Le EP-A-0 515 319 décrit la manière de recueillir un signal d'accélération endocardiaque au moyen d'une sonde endocavitaire pourvue d'une électrode distale de stimulation implantée au fond du ventricule, intégrant en outre un micro-accéléromètre permettant de mesurer l'accélération endocardiaque. Le EP-A-0 655 260 décrit une manière de traiter le signal d'accélération endocardiaque mesuré pour en dériver, notamment, les deux valeurs de pic d'accélération endocardiaque correspondant aux deux bruits majeurs qu'il est possible de reconnaître dans chaque cycle d'un coeur sain.

Plus précisément, le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule et peuvent donc constituer un paramètre représentatif de la contractilité du myocarde. L'amplitude du pic PEA 1 est, plus précisément, corrélée au maximum positif de la variation de pression dP/dt dans le ventricule gauche.

Le second pic d'accélération endocardiaque ("PEA II"), quant à lui, correspond à la fermeture des valvules aortique et pulmonaire au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole.

En variante, le capteur hémodynamique 56 peut être un capteur d'impédance endocavitaire, par exemple un capteur de mesure de bioimpédance (BioZ), comme celui divulgué par exemple dans les EP-A 1 116 497 ou EP-A 1 138 346 (tous deux au nom d'ELA Medical).

Le EP-A-1 116 497 décrit une manière d'effectuer une mesure dynamique de bioimpédance permettant d'évaluer les volumes diastolique et systolique, et d'obtenir ainsi une indication du débit cardiaque et donc de la fraction d'éjection. Ce document décrit plus précisément une technique de mesure de la bioimpédance transvalvulaire (entre oreillette et ventricule situés d'un même côté du coeur) par une configuration tripolaire, avec injection d'une impulsion de courant entre un site atrial et un site ventriculaire, et recueil d'un potentiel différentiel entre un site atrial et un site ventriculaire, avec l'un des sites commun à l'injection et au recueil, un site propre d'injection et un site propre de recueil. Le courant injecté est un courant de faible amplitude, insuffisant pour exciter les cellules cardiaques.

Le EP-A-1 138 346 décrit un autre type de mesure de bioimpédance, qui est une bioimpédance transseptale, c'est-à-dire entre un site situé d'un côté du coeur et un site situé de l'autre côté du coeur. Cette technique permet encore de délivrer une valeur représentative de la fraction d'éjection, bien que le signal soit plus faible que dans le cas de la mesure d'une bioimpédance transvalvulaire, et soit également influencé par l'impédance propre des tissus du septum.

L'analyse du signal de contractilité cardiaque délivré par le capteur 56 peut être effectué de la manière suivante (on prendra l'exemple d'un capteur PEA, mais ces enseignements sont transposables aux signaux délivrés par un capteur de bioimpédance intracardiaque).

Le dispositif relève quotidiennement les points remarquables du signal, par exemple le délai séparant le pic PEA 1 du pic PEA II et les maxima d'amplitude des pics PEA 1 et PEA II. Ces données sont enregistrées dans les mémoires de l'appareil et traitées quotidiennement (filtrage, etc.). Il est avantageux d'opérer un lissage sur le long terme (typiquement, sur une semaine) des amplitudes du PEA pour éviter les variations erratiques ; le critère de déclenchement de l'alerte peut être par exemple du type consistant à comparer la moyenne hebdomadaire des valeurs d'amplitude du PEA 1 à un seuil donné tel que :

$$(1/7)\sum_{i=j-7}^{i=j} (Ampli\_PEA1_{(i)}) < Limite\_delai,$$

l'alerte étant déclenchée lorsque le seuil est franchi.

Une autre possibilité consiste à comparer la moyenne des amplitudes PEA I sur les sept derniers jours par exemple à la moyenne correspondante sur les 21 derniers jours. Si la moyenne sur sept jours est inférieure à un certain pourcentage de la moyenne des 21 derniers jours :

$$(1/7)\sum_{i=j-7}^{i=j} (Ampli\_PEA1_{(i)}) < Pourcent\_Ampli*(1/21)\sum_{i=j-21}^{i=j} (Ampli\_PEA1_{(i)})$$

alors une alerte est déclenchée.

Dans les exemples ci-dessus, les paramètres *Limite_délai* ou *Pourcent_Ampli* correspondent à la « batterie de critères de contractilité cardiaque », avec (comme dans le cas de la batterie de critère d'état clinique) la possibilité de définir au moins deux batteries de critères offrant chacune une sensibilité et une spécificité différentes.

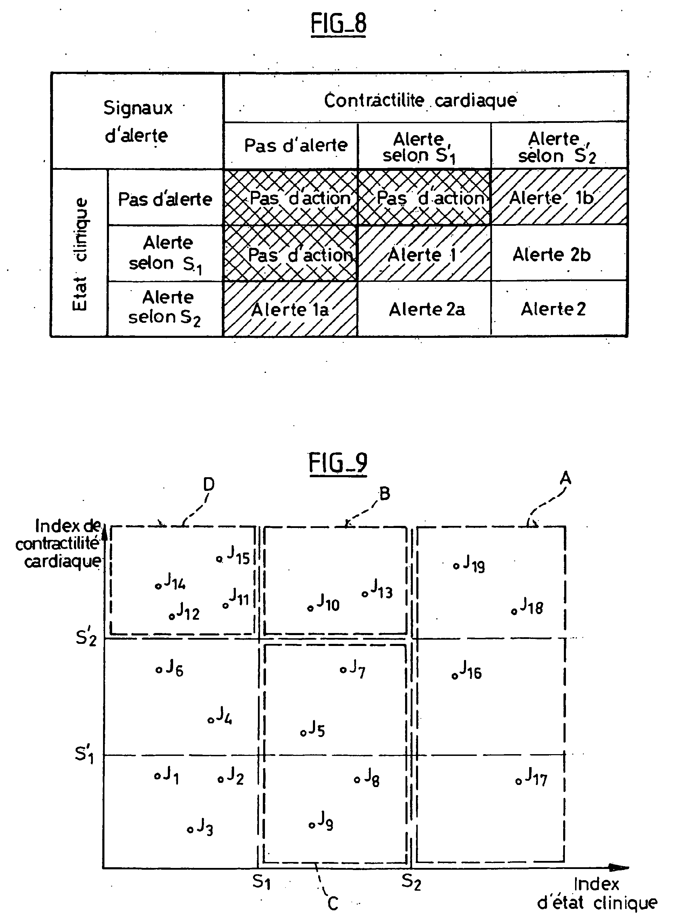

La mise en oeuvre des fonctions de transfert 50 et 54 produit ainsi, par application des batteries de critères {S1, S2} et {S'1, S'2}, quatre possibilités (signaux binaires alerte/pas d'alerte), qui sont combinées entre elles dans le bloc 60 conformément à la table de vérité illustrée figure 8 pour donner en sortie un signal d'alerte composite.

Avantageusement, l'alerte composite produite (ou non) par les moyens d'analyse croisés 60 est une alerte à plusieurs niveaux « pas d'action », « alerte niveau 1 » ou « alerte niveau 2 ». En outre, lorsqu'une alerte est donnée, celle-ci peut être différenciée en sous-niveaux de manière à indiquer de manière plus fine la cause du déclenchement de l'alerte.

Ainsi :

- le cas « pas d'action » correspond à une absence d'alerte à la fois en sortie de la fonction de transfert 50 d'analyse de l'état clinique et de la fonction de transfert 54 d'analyse de la contractilité cardiaque. Dans ce cas, le dispositif ne délivre bien entendu aucune alerte ; il se contente d'enregistrer les données journalières. Il en sera de même lorsque l'une des fonctions de transfert 50 ou 54 ne produit pas d'alerte et que l'autre produit une alerte, mais seulement sur la base de la batterie de critères S1 (ou S'1, respectivement), c'est-à-dire la batterie de critères de sensibilité maximale. Dans ce cas, on peut raisonnablement suspecter un faux positif et éviter alors de déclencher inutilement une alerte.
- le cas « alerte niveau 1 » correspond à une situation où les fonctions de transfert 50 et 54 délivrent toutes deux une alerte sur la base des batteries de critères S1 et S'1 (critères sensibles mais peu spécifiques), ou bien si l'une des fonctions de transfert 50 ou 54 ne produit pas d'alerte mais que l'autre produit une alerte sur la base de la batterie de critères S2 (ou S'2, respectivement), c'est-à-dire de la batterie de critères à forte spécificité. On peut dans ces cas-là suspecter une décompensation avec un impact quotidien sur le patient. L'alerte à ce niveau entraînera par exemple l'envoi automatique d'un message au praticien pour lui demander de prendre contact avec le patient suite à une dégradation importante de son état quotidien du patient pour une raison autre que la décompensation cardiaque (cas de l'« alerte 1a» de la table de vérité de la figure 8), ou bien l'émission d'une alerte en direction du patient pour lui signaler qu'il convient de retourner d'urgence au contrôle médical en raison d'une décompensation cardiaque avancée (« alerte 1 b »).
- le cas « alerte de niveau 2 », plus grave, correspond à une situation où les fonctions de transfert 50 et 54 ont toutes deux produit des alertes, sur la base des batteries de critères S2 et S'2. Il convient d'alerter immédiatement le patient, par exemple par activation d'un vibreur, pour retour d'urgence au contrôle médical en raison d'une décompensation cardiaque avancée. Une alerte de niveau 2 est également produite lorsque l'une des fonctions de transfert a délivré une alerte sur la base d'un critère à forte spécificité S2 (ou S'2, respectivement), l'autre fonction de transfert ayant produit l'alerte seulement sur le critère à forte sensibilité S'1 (ou S1, respectivement). Le patient doit alors être alerté pour retour d'urgence au contrôle médical en raison d'un début de décompensation cardiaque, avec impact quotidien sur son activité (cas de l'« alerte 2a ») ou sans impact quotidien sur son activité (cas de l'« alerte 2b »).

La figure 9 illustre, dans un espace à deux dimensions, la position des index respectifs d'état clinique et de contractilité cardiaque calculés quotidiennement, par rapport aux batteries de critères {S1, S2}, et {S'1, S'2}. Dans cet espace, une ordonnée ou une abscisse croissante indique une spécificité supérieure, avec corrélativement une moindre sensibilité.

Si l'on s'en réfère uniquement à une analyse de l'état clinique (fonction de transfert 50 à partir des signaux physiologique MV et physique G), seules les situations correspondant aux points situés dans la zone A sont susceptibles de donner lieu à une alerte. Pour pouvoir obtenir une alerte avec un point situé dans la zone B, il aurait fallu abaisser de S2 à S1 la spécificité du seuil de détection, avec corrélativement déclenchement d'alertes pour les points situés dans la zone C, alertes correspondant vraisemblablement à des faux positifs. Enfin, un point situé dans la zone D ne serait jamais diagnostiqué comme susceptible d'entraîner une alerte, correspondant ainsi à un faux négatif.

L'analyse combinée de l'état clinique et de la contractilité cardiaque avec, dans chaque cas, application d'au moins deux critères différents, permet de déclencher des alertes pour des points situés dans les zones B et D, sans en déclencher pour ceux situés dans la zone C.

En variante ou en complément, il est possible d'utiliser pour le traitement des données d'état clinique et/ou de contractilité cardiaque un processus de type "machine à états", où les résultats des comparaisons aux divers seuils sont appliqués à un système à transition d'états et à mémoire, qui prend la décision de déclencher une alerte en fonction d'un schéma d'évolution plus complexe. D'autres types d'analyses, plus complexes, peuvent également être mises en oeuvre pour améliorer encore la qualité du procédé de détection, par exemple des techniques de corrélation, d'analyse de morphologie du signal, d'analyse fréquentielle, d'analyse par ondelettes, etc.

## Revendications

1. Un dispositif médical implantable actif, notamment un dispositif de stimulation, resynchronisation, défibrillation et/ou cardioversion, ou un implant actif à visée diagnostique comportant des moyens de diagnostic de l'insuffisance cardiaque,

   dans lequel lesdits moyens de diagnostic de l'insuffisance cardiaque comprennent :

   - des moyens (10, 12) de mesure d'un paramètre représentatif des besoins métaboliques du patient, notamment la ventilation-minute (MV), délivrant un signal de besoin métabolique,
   - des moyens (14, 16) de mesure d'un paramètre corporel à prépondérance physique, notamment l'accélération (G), délivrant un signal physique,
   - des moyens (18) discriminateurs des phases d'activité et de repos, opérant en réponse au signal de besoin métabolique et au signal physique et délivrant un indicateur d'activité du patient (20), et
   - des moyens (50) d'évaluation de l'état clinique du patient, aptes à délivrer au moins un index d'état clinique par application d'au moins une batterie de critères d'état (S1 ; S2) au signal de besoin métabolique, au signal physique et à l'indicateur d'activité,

   dispositif **caractérisé en ce qu'**il comprend en outre :

   - des moyens (56) de mesure d'un paramètre hémodynamique, délivrant un signal hémodynamique représentatif de la contractilité du myocarde,
   - des moyens (54) d'évaluation de la contractilité cardiaque, aptes à délivrer au moins un index de contractilité cardiaque par application d'au moins une batterie de critères de contractilité (S'1 ; S'2) au signal hémodynamique, et
   - des moyens d'analyse croisée (60), recevant en entrée l'(les) index d'état clinique et l'(les) index de contractilité cardiaque, et délivrant en sortie un signal composite d'alerte préventive fonction des valeurs respectives prises par les index d'état clinique et de contractilité cardiaque.

2. Le dispositif de la revendication 1, dans lequel les moyens (56) de mesure d'un paramètre hémodynamique comprennent des moyens de mesure d'une accélération endocardiaque (PEA).

3. Le dispositif de la revendication 1, dans lequel les moyens (56) de mesure d'un paramètre hémodynamique comprennent des moyens de mesure d'une impédance intracardiaque.

4. Le dispositif de la revendication 1, dans lequel les moyens d'analyse croisée (60) comprennent des moyens booléens mettant en oeuvre une table de vérité préenregistrée donnant de manière univoque, pour chaque combinaison possible d'index d'état clinique et de contractilité cardiaque, une valeur correspondante du signal composite d'alerte préventive.

5. Le dispositif de la revendication 1, dans lequel les moyens (50, 54) d'évaluation de l'état clinique et de la contractilité cardiaque sont des moyens aptes à appliquer concurremment au moins deux batteries respectives de critères (S1, S2 ; S'1, S'2), ces deux batteries de critères comprenant une batterie de critères à sensibilité élevée (S1, S'1) et une batterie de critères à spécificité élevée (S2, S'2).

6. Le dispositif de la revendication 5, dans lequel les index d'état clinique et de contractilité cardiaque sont des index booléens susceptibles de prendre trois valeurs, avec une valeur « pas d'alerte » lorsqu'aucune des batteries de critères n'est vérifiée, une valeur « alerte premier niveau » lorsque la batterie de critères à sensibilité élevée est vérifiée, et une valeur « alerte second niveau » lorsque la batterie de critères à spécificité élevée est vérifiée.

7. Le dispositif de la revendication 6, dans lequel le signal composite d'alerte préventive est un signal booléen susceptible de prendre une valeur « pas d'alerte » lorsque :

- les index d'état clinique et de contractilité cardiaque sont tous deux à la valeur « pas d'alerte »,
- l'index d'état clinique est à la valeur « alerte premier niveau » et l'index de contractilité cardiaque est à la valeur « pas d'alerte », ou
- l'index d'état clinique est à la valeur « pas d'alerte » et l'index de contractilité cardiaque est à la valeur « alerte premier niveau ».

8. Le dispositif de la revendication 6, dans lequel le signal composite d'alerte préventive est un signal booléen susceptible de prendre une valeur « alerte premier niveau » lorsque :

- les index d'état clinique et de contractilité cardiaque sont tous deux à la valeur « alerte premier niveau »,
- l'index d'état clinique est à la valeur « alerte second niveau » et l'index de contractilité cardiaque est à la valeur « pas d'alerte », ou
- l'index d'état clinique est à la valeur « pas d'alerte » et l'index de contractilité cardiaque est à la valeur « alerte second niveau ».

9. Le dispositif de la revendication 6, dans lequel le signal composite d'alerte préventive est un signal booléen susceptible de prendre une valeur « alerte second niveau » lorsque :

- les index d'état clinique et de contractilité cardiaque sont tous deux à la valeur « alerte second niveau »,
- l'index d'état clinique est à la valeur « alerte second niveau » et l'index de contractilité cardiaque est à la valeur « alerte premier niveau », ou
- l'index d'état clinique est à la valeur « alerte premier niveau » et l'index de contractilité cardiaque est à la valeur « alerte second niveau ».

10. Le dispositif de la revendication 1, dans lequel les moyens (50) d'évaluation de l'état clinique sont également aptes à évaluer au moins un méta-index (36, 38) à partir des valeurs successives prises par l'index d'état clinique, et appliquer la (les) batterie(s) correspondante(s) de critères au(x)dit(s) méta-index.

11. Le dispositif de la revendication 1, dans lequel lesdits index d'état clinique et de contractilité cardiaque sont évalués à périodicité quotidienne.

**Claims**

1. An active implantable medical device, in particular a pacing, resynchronization, defibrillation and/or cardioversion device, or a diagnosis-directed active implant, including heart failure diagnosis means, wherein said heart failure diagnosis means include :

- means (10, 12) for measuring a parameter, in particular minute ventilation (MV), representative of the metabolic needs of the patient, providing a metabolic need signal;
- means (14, 16) for measuring a predominantly physical body parameter, in particular acceleration (G), providing a physical signal;

- means (18) for discriminating between activity and rest phases, operative in response to the metabolic need signal and to the physical signal, and providing a patient activity index (20); and

- means (50) for evaluating the clinical status of the patient, adapted to provide at least one clinical status index by applying at least one set of status criteria (S1; S2) to the metabolic need signal, to the physical signal, and to the activity index;

said device being **characterized by** further comprising:

- means (56) for measuring a hemodynamic parameter, providing a hemodynamic signal representative of the patient's myocardium contractility,

- means (54) for evaluating cardiac contractility, adapted to provide at least one cardiac contractility index by applying at least one set of contractility criteria (S'1;S'2) to the hemodynamic signal; and

- means (60) for cross-analysis, receiving as input the clinical status index(es) and the cardiac contractility index (es), and providing as output a composite preventive alert signal that is a function of the respective values of the clinical status and cardiac contractility indexes.

2. The device of claim 1, wherein the means (56) for measuring a hemodynamic parameter comprises means for measuring an endocardial acceleration (PEA).

3. The device of claim 1, wherein the means (56) for measuring a hemodynamic parameter comprises means for measuring an intracardiac impedance.

4. The device of claim 1, wherein the means (60) for cross-analysis comprises Boolean means implementing a pre-recorded truth table univalently providing, for each possible combination of clinical status and contractility indexes, a corresponding value for the composite preventive alert signal.

5. The device of claim 1, wherein the means (50, 54) for evaluating clinical status and cardiac contractility are means for concurrently applying at least two respective sets of criteria (S1, S2; S'1, S'2), said two sets of criteria including a set of high sensitivity criteria (S1, S'1) and a set of high specificity criteria (S2, S'2).

6. The device of claim 5, wherein the clinical status and cardiac contractility indexes are Boolean indexes likely to take three values, namely a "no alert" value when no set of criteria is fulfilled, a "first-level alert" value when the set of high sensitivity criteria is fulfilled, and a "second-level alert" value when the set of high specificity criteria is fulfilled.

7. The device of claim 6, wherein the composite preventive alert signal is a Boolean signal likely to take a "no alert" value when:

- the clinical status and cardiac contractility indexes are both set to the "no alert" value,
- the clinical status index is set to the "first-level alert" value and the cardiac contractility index is set to the "no alert" value, or
- the clinical status index is set to the "no alert" value and the cardiac contractility index is set to the "first-level alert" value.

8. The device of claim 6, wherein the composite preventive alert signal is a Boolean signal likely to take a "first-level alert" value when:

- the clinical status and cardiac contractility indexes are both set to the "first-level alert" value,
- the clinical status index is set to the "second-level value" and the cardiac contractility index is set to the "no alert" value, or
- the clinical status index is set to the "no alert" value and the cardiac contractility index is set to the "second-level alert" value.

9. The device of claim 6, wherein the composite preventive alert signal is a Boolean signal likely to take a "second-level alert" when:

- the clinical status and cardiac contractility indexes are both set to the "second-level alert" value,
- the clinical status index is set to the "second-level alert" value and the cardiac contractility index is set to the "first-level alert" value, or
- the clinical status index is set to the "first-level alert" value and the cardiac contractility index is set to the "second-level alert" value.

**10.** The device of claim 1, wherein the means (50) for evaluating the clinical status are further adapted to evaluate at least one meta-index (36, 38) from the successive values taken by the clinical status index, and to apply the corresponding set(s) of criteria to said meta-index(es).

**11.** The device of claim 1, wherein said clinical status and cardiac contractility indexes are evaluated on a daily periodicity.

**Patentansprüche**

**1.** Aktive implantierbare medizinische Vorrichtung, insbesondere eine Vorrichtung zur Stimulation, Re-Synchronisation, Defibrillierung und/oder Kardioversion oder ein aktives Implantat zur Diagnostik, welches Diagnosemittel für Herzinsuffizienz umfasst,
in welcher die Diagnosemittel der Herzinsuffizienz umfassen:

- Messmittel (10, 12) eines Parameters, welcher repräsentativ für die metabolischen Bedürfnisse des Patienten sind, insbesondere die Minutenatmung (MV), welche ein Signal für metabolische Bedürfnisse liefert,
- Messmittel (14, 16) zur Messung eines Körperparameters mit physikalischer Vorherrschaft, insbesondere der Beschleunigung (G), welche ein physikalisches Signal liefert,
- Diskriminatorimittel (18) der Aktivitäts- und Ruhephasen, welche in Antwort auf das Signal metabolischer Bedürfnisse und auf das physikalische Signal arbeiten und ein Aktivitätsindikator des Patienten (20) liefern, und
- Auswertmittel (50) des klinischen Zustands des Patienten, geeignet, um zumindest einen Index des klinischen Zustands durch Anwendung eines Satzes von Zustandkriterien (S1; S2) auf das metabolische Bedürfnissignal, das physikalische Signal und den Aktivitätsindikator,

wobei die Vorrichtung **dadurch gekennzeichnet ist, dass** sie außerdem umfasst:

- Messmittel eines hemodynamischen Parameters (56), welcher ein hemodynamisches Signal liefert, welches repräsentativ für die Kontraktivität des Herzmuskels ist,
- Auswertmittel (54) der Herzkontraktivität, geeignet, um zumindest ein Herzkontraktivitätsindex durch Anwendung zumindest eines Satzes von Kontraktivitätskriterien (S'1; S'2) für das hemodynamische Signal zu liefern, und
- verschränkte Analysemittel (60), welche am Eingang den/die Index(e) des klinischen Zustands und des/der Index(e) der Herzkontraktivität empfangen und am Ausgang ein Kompositsignal zur präventiven Warnung in Abhängigkeit der jeweiligen Werte, welche durch die Indexe des klinischen Zustands und der Herzkontraktivität aufgenommen werden.

**2.** Vorrichtung nach Anspruch 1, in welcher die Messmittel (56) eines hemodynamischen Parameters Mittel zur Messung einer endokardialen Beschleunigung (PEA) umfassen.

**3.** Vorrichtung nach Anspruch 1, in welcher die Messmittel (56) eines hemodynamischen Parameters Mittel zur Messung einer intrakardialen Impedanz umfassen.

**4.** Vorrichtung nach Anspruch 1, in welcher die verschränkten Analysemittel (60) boolesche Mittel umfassen, welche eine Wahrheitstabelle einsetzen, die vorgespeichert ist und in eindeutiger Weise für jede mögliche Kombination des Index des klinischen Zustands und der kardialen Kontraktivität einen jeweiligen Wert des Kompositwarnsignals zur präventiven Warnung ausgibt.

**5.** Vorrichtung nach Anspruch 1, in welcher die Mittel (50, 54) zur Auswertung des klinischen Zustands und der Herzkontraktivität Mittel sind, die geeignet sind, zum gleichzeitigen Anwenden zumindest zweier jeweiliger Sätze von Kriterien (S1, S2; S'1, S'2), wobei diese beiden Sätze von Kriterien einen Satz von Kriterien erhöhter Empfindlichkeit (S1, S'1) und ein Satz von Kriterien mit erhöhter Spezifität (S2, S'2) umfasst.

**6.** Vorrichtung nach Anspruch 5, in welcher die Indexe des klinischen Zustands und der Herzkontraktivität boolesche Indexe sind, die geeignet sind, um drei Werte anzunehmen, wobei ein Wert "keine Warnung", wenn keiner der Kriteriensätze erfüllt ist, ein Wert "Warnung" ersten Grades", wenn der Satz von Kriterien für erhöhte Empfindlichkeit erfüllt ist, und ein Wert "Warnung zweiten Grades" ist, wenn der Kriteriensatz für erhöhte Spezifität erfüllt ist.

**7.** Vorrichtung nach Anspruch 6, in welcher das Warnsignal ein boolesches Signal ist, geeignet um einen Wert "keine Warnung" anzunehmen, wenn:

- die Indexe des klinischen Zustands und der Herzkontraktivtät beide den Wert "keine Warnung" haben,
- der Index des klinischen Zustands auf dem Wert "Warnung ersten Grades" ist und der Index der Herzkontraktivität bei dem Wert "keine Warnung" ist, oder
- der Index des klinischen Zustands beim Wert "keine Warnung" ist und der Index der Herzkontraktivität bei dem Wert "Warnung ersten Grades" ist.

8. Vorrichtung nach Anspruch 6, in welcher das Kompositsignal zur präventiven Warnung ein boolesches Signal ist, geeignet um einen Wert "Warnung ersten Grades" einzunehmen, wenn:

- die Indexe des klinischen Zustands und der Herzkontraktivität beide beim Wert "Warnung ersten Grades" sind,
- der Index des klinischen Zustands beim Wert "Warnung" ersten Grades" und der Index der Herzkontraktivität beim Wert "keine Warnung" ist, oder
- wenn der Index des klinischen Zustands beim Wert "keine Warnung" und der Index der Herzkontraktivität beim Wert "Warnung zweiten Grades" ist.

9. Vorrichtung nach Anspruch 6, in welcher das Kompositsignal zur präventiven Warnung ein boolesches Signal ist, geeignet um einen Wert "Warnung zweiten Grades" einzunehmen, wenn:

- die Indexe des klinischen Zustands und der Herzkontraktivität beide beim Wert "Warnung zweiten Grades" sind,
- der Index des klinischen Zustands beim Wert "Warnung zweiten Grades" ist und der Herzkontraktivitätindex beim Wert "Warnung ersten Grades" ist, oder
- wenn der Index des klinischen Zustands beim Wert "Warnung ersten Grades" und der Index der Herzkontraktivität beim Wert "Warnung zweiten Grades" ist.

10. Vorrichtung nach Anspruch 1, in welcher die Mittel (50) zur Auswertung des klinischen Zustands auch geeignet sind, um zumindest einen Metaindex (36, 38) ausgehend von voneinander folgenden Werten, die durch den Index des klinischen Zustands aufgenommen wurden, auszuwerten und den(die) jeweiligen Satz(Sätze) von Kriterien auf den(die) Metaindex(e) anzuwenden.

11. Vorrichtung nach Anspruch 1, in welcher die Indexe des klinischen Zustands und Herzkontraktivität mit täglicher Periodizität ausgewertet werden.

## FIG_1

10 — (MV)　　　　　　　　(G) — 14

12 — | _amplitude<br>_période |　　MV　　G　　| Accélération<br>(Gi) | — 16

| Algorithme double capteur<br>discrimination phases<br>activité / repos | — 18

| Indicateur<br>activité/repos | — 20

Activité　　　　　　　　　　Repos

22 — | Mémorisation:<br>_ Tact<br>_ $\overline{\Sigma Gi}$<br>_ $\overline{MV_{act}}$ |　　| Mémorisation:<br>_ Trep<br>_ $\overline{MV_{rep}}$ | — 24

| Analyse sur 24h<br>(application de règles) | — 26

28 — | Index<br>G |　　30 — | Index<br>$MV_{act}$ |　　32 — | Index<br>$MV_{rep}$ |　---- 

| Analyse rétrospective sur plusieurs jours<br>(application de méta-règles) | — 34

36 — | Méta-<br>index1 |　　38 — | Méta-<br>index2 |　----

| Déclenchement éventuel d'une<br>alerte préventive | — 40

## FIG_2

## FIG_3

## FIG_4

## FIG_5

FIG_6

Spécificité

100% $S_2(S_2')$

$S_1(S_1')$

100%

Sensibilité

FIG_7

10
MV

14
G

56
PEA (ou BioZ)

Etat
clinique

∫

50

52
$S_1, S_2$

58
$S_1', S_2'$

∫

Contractilité
cardiaque

54

alerte/pas d'alerte          alerte/pas d'alerte

60
⊗

- Alerte niveau 1
- Alerte niveau 2
- Pas d'alerte

## FIG_8

| Signaux d'alerte | | Contractilite cardiaque | | |
|---|---|---|---|---|
| | | Pas d'alerte | Alerte, selon $S_1$ | Alerte, selon $S_2$ |
| Etat clinique | Pas d'alerte | Pas d'action | Pas d'action | Alerte 1b |
| | Alerte selon $S_1$ | Pas d'action | Alerte 1 | Alerte 2b |
| | Alerte selon $S_2$ | Alerte 1a | Alerte 2a | Alerte 2 |

## FIG_9

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0750920 A
- EP 0919255 A
- EP 1533001 A
- US 2003040776 A
- EP 0966987 A
- EP 0515319 A
- EP 0582162 A
- EP 0655260 A
- EP 1116497 A
- EP 1138346 A